# FASCICULE DE BREVET EUROPEEN

(11) **EP 0 435 713 B1**
(45) Date de publication et mention de la délivrance du brevet: **25.05.1994**
(21) Numéro de dépôt: 90403415.4
(22) Date de dépôt: 30.11.1990
(51) Int. Cl.: E21B 49/00, G01N 33/28, G01N 11/00

(54) **Dispositif pour étudier le vieillissement d'un fluide en circulation dans des conditions spécifiques imposées**
Vorrichtung zur Untersuchung der Alterung einer Flüssigkeit unter kontrollierten Bedingungen
Apparatus to study the ageing of a circulating fluid under controlled conditions

(30) Priorité: 20.12.1989 FR 8917030
(43) Date de publication de la demande: 03.07.1991
(73) Titulaire: INSTITUT FRANCAIS DU PETROLE, 92502 Rueil-Malmaison (FR)
(72) Inventeur: Degouy, Didier, F-78800 Houilles (FR); Brandely, José, F-91600 Savigny S/Orge (FR); Chatelain, Bernard, F-95000 Jouy Le Moutier (FR); Gonzalez, Pierre, F-92500 Rueil-Malmaison (FR)

(56) Documents cités:
- US-A- 4 501 143
- US-A- 4 586 376
- US-A- 4 790 933

## Description

La présente invention concerne un dispositif pour étudier de manière accélérée le vieillissement d'un fluide en circulation, dans des conditions spécifiques imposées.

Avec le dispositif selon l'invention, on peut effectuer rapidement deux types d'études différentes. Il peut être utilisé pour évaluer les qualités particulières d'une formule de fluide, dans le cadre d'opérations de qualification de ce fluide pour un usage particulier ou bien encore pour déterminer les lois de comportement dans le temps d'un certain fluide lorsqu'il est soumis à des conditions de fonctionnement reproduisant les conditions extrêmes rencontrées en opération.

Le dispositif selon l'invention peut notamment servir pour étudier et tester des boues comme on en utilise au cours d'opérations de forage de puits tels que des puits de forage pétroliers.

Durant les opérations de forages pétroliers, une circulation de boue sous pression est établie entre l'installation de surface et la zone environnant l'outil de forage. La boue sert à refroidir et lubrifier l'outil de forage ainsi qu'à nettoyer le trou foré en transportant les déblais jusqu'à la surface. Elle sert aussi à créer une pression hydrostatique suffisante pour stabiliser les parois du puits et contenir les fluides sous pression dans les formations environnant le forage. La boue est donc à son passage dans les zones forées, soumise à des contraintes importantes dépendant des caractéristiques des puits, qu'il s'agisse de températures très élevées, jusqu'à 200°C, de pressions élevées de l'ordre de plusieurs dizaines de MPa et de cisaillements au niveau de l'outil de forage. Il est fréquent que les conditions de forage, la température et la nature des formations changent. Ces changements peuvent affecter les propriétés du fluide de forage. Il est donc important de s'assurer que le fluide employé possède les propriétés qui conviennent et les conserve au cours du temps malgré les contraintes subies.

L'étude du comportement des fluides de forage peut être effectuée de deux manières différentes.

On peut inclure différents appareils de mesure dans l'installation de pompage délivrant le fluide sous pression, pour tester en continu les caractéristiques du fluide remontant de la zone de forage. Un appareil permettant de faire des tests de ce genre est décrit par exemple dans le brevet U.S No. 4 635 735. Ce type de mesures présente l'avantage que le fluide étudié est soumis aux conditions mêmes régnant au fond d'un forage. Sa mise en oeuvre nécessite cependant que l'on modifie et adapte une installation existante de circulation de fluide de forage, ce qui n'est pas toujours possible.

On peut aussi choisir d'étudier en laboratoire le comportement d'un fluide. Dans une installation in situ, les cycles de mesures sont liés aux cycles de circulation de la boue de forage. Le rythme de circulation étant imposé et relativement lent, l'étude de l'évolution du fluide au cours du temps est généralement très longue. Avec un appareil de laboratoire au contraire, on peut simuler les conditions régnant dans une zone de forage en ce qui concerne des paramètres bien définis en imposant un rythme de circulation du fluide beaucoup plus rapide. Par ce moyen, le fluide subit un vieillissement accéléré. Des tests autrement très longs, sont menés à bien rapidement. En outre, la possibilité de faire varier à loisir certaines conditions expérimentales reproduisant les variations observées au cours des forages, permet de multiplier les tests et les mesures. Différents appareils de ce type sont décrits dans les brevets U.S No. 4 483 189, 4 501 143 ou 4 510 800.

Par le brevet US-A-4 790 933, on connait un dispositif pour faire des mesures sur des caractéristiques de boues de forage comportant une boucle d'essai où l'on fait circuler la boue au moyen d'une pompe de ciruclation, pourvue de moyens de chauffage, de moyens de mesure de caractéristiques rhéologiques et de moyens de filtration.

La difficulté à résoudre quand on veut réaliser un appareil de simulation du fluide est de bien contrôler les paramètres agissant sur le fluide étudié tout le long de chaque cycle de travail imposé, en évitant au mieux les variations incontrôlées des paramètres et plus généralement tout ce qui est susceptible de fausser les mesures. Les fluides de forage contiennent souvent des substances corrosives (électrolytes) où abrasives (sulfate de baryum - argiles) qui du fait des conditions d'essais (température - circulation) sont susceptibles d'attaquer les matériaux constitutifs des boucles de circulation. Le fluide peut alors se trouver contaminé involontairement par des ions métalliques qui faussent les résultats des mesures. Dans les boucles de circulation existantes, le chauffage du fluide est réalisé le plus souvent en un emplacement unique. Pour tenir compte des limitations de certains appareillages inclus dans la boucle, on est obligé aussi soit de refroidir le fluide soit de compliquer l'appareillage. Les conditions expérimentales réalisées par les boucles de simulation ne reflètent pas au mieux de ce fait les conditions réelles régnant dans le fond des puits.

On doit aussi noter que les appareillages d'étude de fluides en circulation deviennent rapidement très complexes si l'on veut obtenir un ensemble complet de mesures. De ce fait, le maintien durant toutes les opérations sur le fluide de la boucle fermée, de conditions expérimentales précises telles que des valeurs de température et de pression devient très difficile à mettre en oeuvre.

Le dispositif selon l'invention permet de réaliser un ensemble de mesures sur un courant de fluide soumis à certaines conditions spécifiques semblables à celles rencontrées au cours de son utilisation circulant dans un circuit fermé en évitant les inconvénients ci-dessus énoncés.

Il comporte une pompe de circulation incluse directement dans le circuit fermé pour établir un courant de fluide dans ledit circuit, des moyens d'asservissement pour réguler la différence de pression entre la sortie et l'entrée de la pompe de circulation, des moyens pour cisailler le fluide circulant dans le circuit fermé, des moyens pour mesurer les débits de fluide dans le circuit fermé, des moyens pour mesurer les caractéristiques rhéologiques du fluide, des moyens de chauffage pour chauffer le fluide en circulation, des moyens de filtration du fluide en circulation. Le dispositif est caractérisé en ce que la pompe de circulation est du type à membranes, les moyens d'asservissement sont agencés pour réguler ladite différence de pression de part et d'autre des membranes de la pompe de circulation, et les moyens de chauffage sont disposés de façon à chauffer le fluide en circulation, de façon homogène, le dispositif comportant également une pompe de chargement pour introduire dans le circuit fermé un ou plusieurs fluides, une pompe de régulation de filtration, les moyens de filtration étant actionnés par celle-ci, une pompe d'injection et de soutirage, des capteurs de mesure, un ensemble d'alimentation électrique et un système de commande programmable connecté aux capteurs de mesure et coopérant avec l'ensemble d'alimentation électrique, pour imposer aux pompes et aux vannes de commande, des séquences opératoires pour la réalisation de l'ensemble de mesures sur le fluide en circulation.

Les moyens de chauffage comportent par exemple un ensemble de manchons chauffants régulés en température.

Les moyens de filtration sont inclus par exemple sur un circuit dérivé, et comportent une pluralité d'unités de filtration à manchons filtrants interconnectées en série, chacune d'elles étant reliée par une vanne à la pompe de régulation de la filtration laquelle applique sélectivement auxdites unités de filtration une pression différentielle de part et d'autre des manchons filtrants pour dériver du fluide hors du circuit fermé.

De préférence, le circuit comporte essentiellement des portions de circuit orientées de manière à éviter la formation de dépôts susceptibles de restreindre localement la section du circuit fermé.

Le système de commande programmable comporte par exemple un automate programmable adapté à centraliser les valeurs des paramètres mesurés associé à un micro-ordinateur de commande pour imposer des valeurs aux paramètres de régulation et des moyens de liaison pour assurer l'intéraction de l'automate et du micro-ordinateur de commande, le dispositif comportant en outre un ensemble de visualisation montrant à chaque instant l'état de ses différents éléments.

Suivant un mode de réalisation préféré, on utilise comme pompe de circulation, une pompe à membranes métalliques connectée directement sur le circuit fermé.

Les moyens d'asservissement comportent par exemple deux capteurs de pression disposés au voisinage respectivement de l'entrée et de la sortie de la pompe de circulation et deux capteurs de pression disposés de part et d'autre des membranes de cette même pompe.

Le dispositif selon l'invention permet de maintenir fidèlement les conditions imposées à un fluide pour les raisons suivantes :
- La répartition des moyens de chauffage tout au long de la boucle fermée permet un chauffage homogène de tout le fluide et évite l'apparition de points chauds et de points froids qui engendrent des problèmes de régulation de pression dans les circuits, et, en ce qui concerne les points chauds, risquent de provoquer une dégradation locale des fluides en circulation.
- La pompe de chargement permet de faciliter la phase de mise en place et la pompe de régulation de filtration, de maintenir plus facilement à une valeur déterminée la différence de pression nécessaire aux fonctionnement des unités de filtration.
- L'utilisation d'une pompe de circulation du type à membranes est en outre très favorable au maintien de la température et de la pression fixées pour le fluide en circulation.
- Avec l'automate programmable utilisé qui impose strictement des séquences opératoires aux différents éléments (vannes, pompes etc) en fonction des mesures fournies par des capteurs et qui contrôle leur évolution, cet automate étant sous le contrôle direct d'un opérateur par le biais d'un micro-ordinateur de commande, on peut facilement réaliser des séquences complexes sans s'écarter de conditions imposées, et donc on facilite l'obtention de résultats représentatifs.

D'autres caractéristiques et avantages du dispositif selon l'invention apparaîtront mieux à la lecture de la description ci-après d'un mode de réalisation décrit à titre d'exemple non limitatif, en se référant aux dessins annexés où :
- la Fig.1 montre de façon schématique l'agencement des circuits de la boucle fermée; et
- la fig. 2 montre un schéma synoptique des éléments de commande et de contrôle agissant sur les circuits de la boucle fermée.

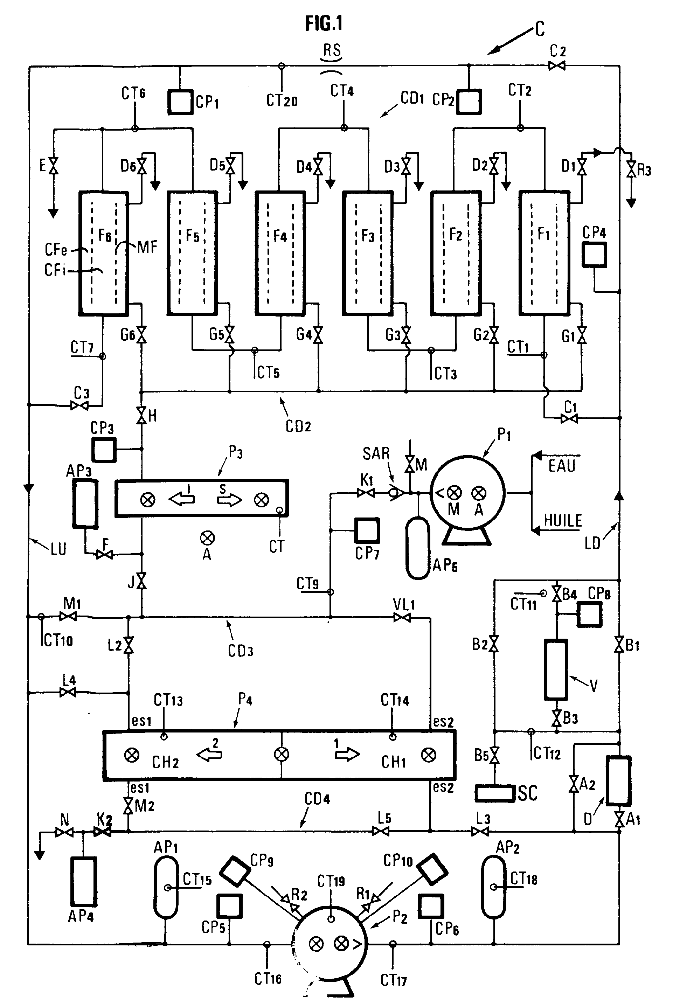

Le dispositif schématisé à la Fig. 1 comporte un circuit fermé ou boucle C apte à imposer à un fluide qui y circule, des conditions précises qui reproduisent celles qu'il subit ou doit subir en opération. Dans le domaine d'application des boues pour forage, le dispositif doit imposer au fluide dans le circuit certains paramètres: température Tc, pression Pc, cisaillement etc, reproduisant celles rencontrées au fond d'un puits au voisinage de l'outil de forage. Le volume du circuit C est choisi relativement faible, de l'ordre d'une dizaine de litres par exemple. Il est réalisé dans un alliage capable de résister à la corrosion par les solutions salines à haute température et à l'abrasion par des solutions chargées en particules solides.

La boucle C est constituée pour l'essentiel de portions de circuit orientées verticalement. Elle est optimisée à cet égard pour réduire le plus possible les portions de circuit de raccordement horizontales ou à faible inclinaison sur l'horizontale. De cette façon a été éliminée toute possibilité de sédimentation en circulation des particules solides que le fluide étudié peut contenir. On évite ainsi les restrictions de section des canalisations dues à des accumulations locales de sédiments et du même coup les effets de cisaillement incontrôlés qu'ils pourraient provoquer sur le fluide en circulation. On simplifie aussi beaucoup la remise en état du dispositif à la fin des essais. La longueur de la boucle est de l'ordre d'une dizaine de mètres par exemple.

La boucle C est fermée sur une pompe de circulation P2 capable de fonctionner sous une pression de plusieurs dizaines de MPa (50 MPa par exemple), en autorisant une pression différentielle de l'ordre d'une dizaine de MPa entre son entrée (aspiration) et sa sortie (refoulement). On utilise de préférence une pompe à membranes métalliques à double effet qui agit directement sur le fluide en circulation sans avoir à modifier sensiblement sa température et sa pression et qui permet d'obtenir une étanchéité parfaite. Ceci est indispensable compte-tenu du faible volume du circuit C car une faible perte de liquide risquerait d'entraîner une chute importante de la pression et par conséquent une vaporisation du fluide. Une parfaite étanchéité garantit en outre qu'aucune compensation de pertes n'est nécessaire et donc que c'est le fluide initialement introduit dans la boucle qui est étudié durant tout l'essai. La pompe est spécialement étudiée pour résister aux fluides chauds, abrasifs et corrosifs. Elle ne provoque aucun refroidissement sensible du fluide. Deux amortisseurs de pulsations AP1 et AP2 communiquent avec le circuit C au voisinage de la pompe P2. Le premier AP1 est disposé en aval de la pompe, l'autre en amont. Leur fonction est d'assurer un débit constant dans le circuit C. La pompe P2 assure un débit de l'ordre de 20 l/min par exemple.

Le débit est mesuré par un débitmètre D connecté sur le circuit par l'intermédiaire d'une vanne A1. Une dérivation contrôlée par une vanne A2, permet de mettre le débitmètre D en ou hors circuit, à volonté. Sur le circuit LD en aval de la pompe P2, et en aval du débitmètre, est placé une vanne B1 et, en parallèle sur celle-ci, un premier élément de circuit comportant un viscosimètre V et deux vannes B3 et B4 de contrôle, et un second élément de circuit contrôlé par une vanne B2. Un circuit de prélèvement contrôlé par une vanne B5 permet de connecter une cellule de prélèvement Sc d'un type connu. Les prélèvements de fluide opérés à différents moments du cycle de vieillissement permettent de suivre l'évolution des propriétés physico-chimiques du fluide: pH, potentiel oxydo-réducteur (redox), d'étudier l'évolution de la structure des argiles et la dégradation des constituants organiques. On peut utilement rapprocher les mesures faites sur les prélèvements, des différentes mesures rhéologiques effectuées au cours du cycle de vieillissement. Le viscosimètre V permet de faire des mesures discontinues, à intervalles de temps déterminés, toutes les heures par exemple.

En aval du débitmètre, la canalisation aval LD du circuit C est pourvue d'une restriction de section RS capable d'imposer au fluide en circulation, à chaque cycle, un taux de cisaillement déterminé en fonction d'une certaine application. Dans le cas d'un fluide de forage, on choisit un rétrécissement qui applique un taux de cisaillement de l'ordre de 10⁴ s⁻¹ ayant le même ordre de grandeur que le taux de cisaillement subi par le fluide au passage dans un outil de forage. L'accès au rétrécissement est contrôlé par une vanne C2. Deux capteurs de pression CP1, CP2 sont disposés sur le circuit C de part et d'autre du rétrécissement RS pour mesurer la perte de charge qu'il provoque.

Du fait de la faible capacité du circuit et du débit de la pompe de circulation P2, le fluide parcourt rapidement la boucle C. On peut ainsi multiplier la fréquence des passages au travers du rétrécissement RS et accélérer le vieillissement du fluide étudié.

Une cellule de filtration est disposée en dérivation sur le rétrécissement RS et la vanne de contrôle C2. Cette cellule de filtration comporte par exemple six unités de filtration F1 à F6 interconnectées en série sur une dérivation commune cd dont l'accès est contrôlé par une vanne C1.

Cette structure de filtres en série est avantageuse. Les chambres des filtres et des vannes peuvent être usinées dans un même bloc, ce qui évite l'utilisation de nombreux corps de vanne et de raccords et, d'une façon générale, le nombre de vannes nécessaires pour le contrôle de la structure de filtration, est moins grand que pour une autre configuration.

Chaque unité de filtration comporte une cavité séparée en deux chambres coaxiales CFi et CFe par un manchon filtrant MF. Quand la pression est la même dans les deux chambres, l'unité de filtration est inactive. Le fluide la traverse en restant au centre du manchon filtrant MF. Pour établir une pression différentielle de filtration dans les unités de filtration et ainsi forcer le fluide à traverser les manchons filtrants, chaque chambre périphérique CFe est reliée par l'intermédiaire d'une vanne de contrôle G1, G2...G6, à une canalisation commune cd2, elle-même reliée par une autre vanne H, à une pompe de régulation de la filtration P3 adaptée à créer et à maintenir une différence de pression △ P bien constante entre la pression P dans le circuit fermé C et la pression P′ régnant dans les chambres annulaires CFe des différentes unités de filtration F1 à F6, pendant toute la durée de la filtration lorsque l'une quelconque d'entre elles est rendue active. Les unités de filtration sont pourvues de circuits de purge contrôlés par des vannes de purge D1, D2...D6. Une vanne de purge E est connectée aux sorties des filtres F5 et F6. La pompe de régulation de la filtration P3 est par exemple du type à piston ou plongeur.

Le fonctionnement de cette pompe P3 est contrôlée par comparaison entre les valeurs mesurées par un capteur de pression CP3 connecté à l'élément de circuit cd2 en aval de la vanne H et un capteur de pression CP4 connecté au circuit C. Un amortisseur de pulsations AP3 est connecté à la pompe P3 par une vanne de contrôle F. Il permet la décharge de la pompe P3 lorsque celle-ci est remplie. Une vanne de contrôle J met la pompe P3 en communication avec un élément de circuit cd3 relié par une vanne M1 avec la canalisation LU du circuit principal C en amont de la pompe de circulation P2, et par deux vannes de contrôle L1 et L2 respectivement avec deux entrées-sorties es1, es2 d'une pompe à piston à double effet P4 permettant l'injection et le soutirage. Une vanne L4 fait communiquer l'entrée es1 avec le circuit amont LU. La pompe P4 a pour fonctions:
- de comprimer le fluide pour l'amener à la pression de service;
- de réguler par soutirage ou par injection la pression du fluide dans le circuit pendant l'essai;
- d'équilibrer la pression du fluide durant les essais, notamment pendant les phases de filtration et de prise d'échantillons;
- d'injecter dans le fluide en circulation, des particules solides en suspension caractéristiques d'un terrain par exemple, ou encore des solutions salines représentant des fluides de formation; et
- d'effectuer des prélèvements de fluides hors du circuit.

Les deux entrées-sorties es1, es2 de la pompe P4 sont connectées par l'intermédiaire de vannes respectivement M1 et L5, avec une portion de circuit cd4 qui communique par l'intermédiaire d'une vanne de contrôle K2 avec un amortisseur de pulsations AP4. L'entrée-sortie es2 d'injection de la pompe P4, communique par une vanne L3 avec le circuit aval LD du circuit principal C du côté de la sortie de la pompe de circulation P2. Une vanne L4 contrôle la communication entre l'entrée-sortie es1 et la canalisation amont LU du circuit principal C du côté de l'entrée de la pompe de circulation P2.

Le chargement du circuit principal en fluide est assuré par une pompe de chargement P1 dont la sortie communique avec un amortisseur de pulsations AP5 et, par l'intermédiaire d'une soupape anti-retour SAR et d'une vanne de contrôle K1, avec l'élément de circuit cd3.

Deux capteurs de pression CP5 et CP6 sont disposés directement en amont et en aval de la pompe P2. La comparaison des indications de ces deux capteurs permet de vérifier que les pertes de charge tout au long du circuit C sont compatibles avec la pression différentielle maximale admissible par la pompe de circulation P2. Un capteur de pression CP7 est connecté sur l'élément de circuit CD3 et ses mesures permettent l'asservissement de la pompe de chargement P3. Si la pression qu'il mesure est inférieure à un seuil bas fixé, la pompe de chargement charge la pompe P4. Si elle est supérieure à un seuil haut, la pompe de chargement P3 s'arrête. Un capteur de pression CP8 est disposé dans le circuit du viscosimètre V pour mesurer la pression dans celui-ci, notamment durant les phases de mesure où il est isolé du reste du circuit. Deux capteurs CP9 et CP10 sont utilisés pour mesurer les pressions de part et d'autre de chaque membrane métallique de la pompe de circulation P2. Leurs mesures sont utilisées pour asservir les pressions de part et d'autre de chaque membrane. On peut ainsi leur éviter des conditions de fonctionnement excessives en cas d'anomalie.

Deux vannes de purge R1, R2 sont disposés dans la pompe de circulation P2. Elles sont utilisées durant les phases de chargement en fluide.

La pression de service est obtenue en partie par échauffement du fluide. Tous les circuits et les éléments constitutifs en contact avec le fluide en circulation (pompes, cellule de filtration, amortisseur de pulsation, viscosimètre etc) sont pourvus d'un gainage isolant. L'appareil comporte un nombre important, une trentaine par exemple, de manchons de chauffage (non représentés) répartis sur tout le circuit principal C et les circuits dérivés, chacun d'eux étant pourvu d'un capteur de température et d'une régulation propre de sa température, du type P.I.D. Ces manchons assurent un chauffage homogène de l'ensemble de la boucle et permettent de porter le fluide tout au long des circuits à une température pouvant atteindre 200°C. Ceci est important car, pendant les phases statiques, il ne doit y avoir ni points chauds ni points froids. En effet, en raison de la mauvaise transmission de pression dans les fluides thixotropes, l'existence de points chauds dans les circuits amenèrait une augmentation locale de pression au-delà des limites de sécurité provoquant un arrêt de fonctionnement, et aussi une dégradation locale éventuelle du fluide. L'existence de points froids pourrait abaisser la pression du fluide en deçà d'un seuil bas fixé et provoquer aussi un arrêt de fonctionnement.

De nombreux capteurs de température et de pression référencés respectivement CT1, CT2...CT20 sont disposés en différents emplacements du circuit principal C et des circuits dérivés, pour mesurer les températures du fluide en circulation.

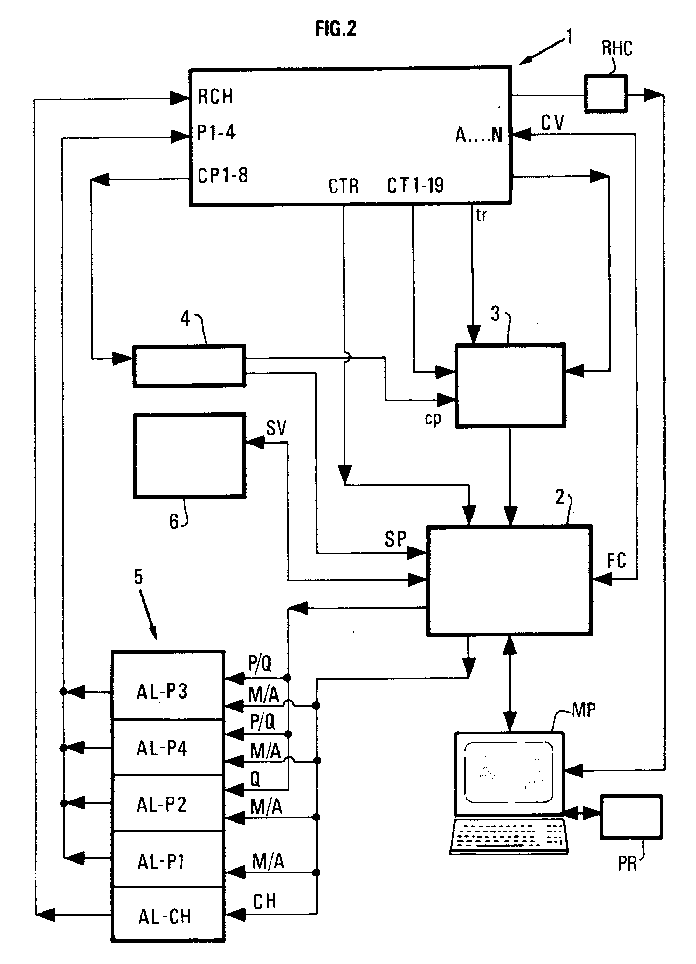

Sur la Fig. 2, le bloc 1 représente l'ensemble des circuits et éléments de la Fig. 1. Un automate programmable 2 d'un type connu, tel qu'un automate M.G PB400 (exemple non limitatif), est utilisé pour gérer l'ensemble des processus intervenant dans le bloc 1. Il est associé à un micro-ordinateur de commande MP. L'automate 2 est couplé avec un ensemble d'acquisition 3 adapté à multiplexer, échantillonner et numériser les différents signaux de température et de pression mesurés dans le bloc 1. L'ensemble d'acquisition 3 reçoit directement les signaux analogiques délivrés par le débitmètre D et les capteurs de température CT1 à CT20 ainsi que les signaux TR délivrés par les capteurs de température associés respectivement aux manchons de chauffage.

Les signaux de pression des capteurs CP1 à CP10 sont appliqués à un ensemble de mesure de pression 4 qui délivre à l'ensemble d'acquisition 3 les valeurs cp des pressions mesurées.

L'automate 2 reçoit les mesures numérisées par l'ensemble d'acquisition 3 et différents signaux de fonctionnement CTR. Il s'agit de signaux provenant du bloc 1: signaux de position Marche/Arrêt des pompes P1 à P4, signaux de fin de course FC reçus des différentes pompes P1 à P4 et des vannes A à N, signaux indiquant la vitesse de rotation de la pompe de circulation P2. Il s'agit encore de signaux provenant d'émetteurs d'impulsions (non représentés) associés aux pompes P3 et P4 qui engendrent des impulsions indicatives de la position exacte de leurs pistons respectifs et qui permettent de contrôler leurs débits d'injection Q respectifs lorsqu'elles sont régulées en débit.

En réponse aux mesures et signaux qu'il reçoit et des consignes imposées par le micro-ordinateur MP, l'automate 3 élabore des ordres. Ceux qui concernent les vannes sont envoyés directement dans le bloc 1.

Suivant les phases et les séquences de fonctionnement qui seront explicitées plus loin, les pompes peuvent être régulées en débit (Q) ou en pression (P). D'autres ordres sont appliqués à un ensemble de puissance 5 comportant cinq unités. Quatre unités ALP1, ALP2, ALP3 et ALP4 reçoivent de l'automate 2 des signaux de marche-arrêt M/A pour les pompes P1 à P4 du bloc 1 d'une part et des valeurs de consigne pour les pompes P2 à P4 selon qu'elles doivent être régulées en pression ou en débit. En réponse à ces signaux, ces unités fournissent respectivement aux pompes les courants nécessaires pour les actionner. Une cinquième unité ALCH commande les relais d'alimentation des manchons de chauffage dans le bloc 1, en réponse à une commande appropriée provenant de l'automate 2 et élaborée à partir des valeurs lues des paramètres de régulation P.I.D..

Le dispositif comporte aussi un écran de visualisation 6 où l'ensemble des circuits, des pompes, des vannes et des capteurs est représenté sous une forme synoptique, et qui montre à chaque instant l'état de ses différents éléments constitutifs. L'écran synoptique 6 reçoit de l'automate 2 les signaux de visualisation SV appropriés. Une imprimante PR est associée au micro-ordinateur MP.

Les phases (en mode automatique) et les séquences (en mode semi-automatique) se déclenchent à partir de l'écran synoptique 6 par affichage sur celui-ci d'un numéro d'ordre et validation de l'opération correspondante. Lorsqu'une phase est terminée en mode automatique, l'automate 2 repositionne la boucle en phase de vieillissement et attend un ordre pour en lancer une autre plus spécifique.

L'automate est programmé pour gérer automatiquement les différentes phases de fonctionnement des circuits du bloc 1 comme on va le voir dans la suite de la description. Cependant, l'opérateur peut intervenir sur le déroulement des processus en cours au moyen du micro-ordinateur de commande MP reliée à l'automate 2. Le micro-ordinateur MP reçoit les mesures transmises par l'automate et en retour, lui transmet des valeurs de consignes. Il reçoit en outre directement les mesures provenant du viscosimètre V par l'intermédiaire d'un ensemble d'interface RHC. Les consignes imposées par le micro-ordinateur MP sont adaptées pour le viscosimètre V par le même ensemble d'interface RHC.

Différents processus de fonctionnement de la boucle C incluse dans le bloc 1, vont être décrits ci-après pour montrer les séquences opératoires qui sont conduites par l'automate 2.

Les différentes phases du fonctionnement de l'appareil selon l'invention seront réalisées par modification de sa conformation, en changeant l'état des pompes et des vannes.

### Remplissage de la boucle en fluide clair (Phase 01)

Le piston de la pompe P4 est placé en position médiane et celui de la pompe P3 en position S.

L'opération de remplissage en fluide clair sera effectuée par la pompe P1 en plusieurs séquences pour limiter au maximum les piégeages d'air (séquences 01 à 04).

### Test de la boucle à la pression de consignes Pc et à température ambiante (Phase 02)

La boucle est tout d'abord testée à la pression de refoulement de la pompe 1 afin de détecter d'éventuelles fuites importantes (séquence 1). La pression de consigne sera ensuite obtenue par injection de fluide de base dans le circuit principal à l'aide de la pompe P4. L'injection se fera alternativement par les deux chambres de la pompe:
. Injection dans le sens 1 (séquence 02)
. Injection dans le sens 2 (séquence 03)

Ces séquences seront gérées par l'automate 2, le changement de séquence étant déterminé par un témoin de fin de course du piston.

L'injection par l'une des chambres entraînera une baisse de pression, mesurée par le capteur CP7, dans l'autre chambre. Le chargement de cette dernière s'effectue automatiquement avec la pompe P1. On évite une trop grande sollicitation de la pompe en l'actionnant ou en l'arrêtant en fonction de la pression p7 mesurée.

Après contrôle de l'étanchéité à la pression de consigne, la boucle est dépressurisée (séquence 04) et de piston de la pompe P4 est amené en butée dans le sens 1.

### Remplissage de la boucle avec du fluide de forage

Cette opération est réalisée en plusieurs phases comprenant chacune plusieurs séquences de façon à obtenir le meilleur balayage possible du fluide clair.

### Remplissage de la pompe d'injection-soutirage (P4): phase 03

Le piston de la pompe est déplacé dans le sens 2 avec remplissage simultané de la chambre 1 par la pompe P1 (séquence 01) puis l'opération est répétée dans l'autre sens (séquence 02). Les séquences 01 et 02 sont doublées.

### Remplissage du reste de la boucle (phase 04)

Il s'effectue en plusieurs étapes ou séquences:
- remplissage de la partie basse de la boucle (séquence 01)
- remplissage des dérivations du débitmètre D et du viscosimètre V et des cinq premiers filtres F1 à F5 (séquence 02)
- remplissage du débitmètre D de la canalisation aval pour ouverture de la vanne B1 et des cinq premiers filtres F1 à F5 (séquence 03)
- remplissage de la canalisation par ouverture de C2 et du sixième filtre F6 (séquence 04)
- remplissage de la canalisation retournant vers la pompe P2 et du sixième filtre (séquence 05).

Il faut noter toutefois que le remplissage en fluide de forage ne concerne pas le circuit CD2 relié aux chambres annulaires des manchons filtrants MF qui ne contient que du fluide clair.

### Remplissage du viscosimètre (phase 05)

Cette opération n'est faite que lorsque le fluide contenu dans la boucle a les caractéristiques souhaitées, traduisant ainsi un déplacement complet du fluide initial. Après réduction du débit de la pompe P1, le viscosimètre V est rempli (séquence 01) puis isolé par fermeture de la vanne B3 et la boucle est testée à une pression de test (séquence 02).

### Mise en conditions de température et de pression

### Début de circulation (phase 06)

La pompe de circulation est mise en marche de façon à homogénéiser la température du fluide dans la boucle pendant cette phase de mise en conditions (séquence 01). La circulation est faite à faible débit car à ce stade, les amortisseurs de pulsations ne peuvent remplir que partiellement leur fonction régulatrice de débit.

### Phases de chauffage et régulation (phases 07 et 08)

Pendant la phase de chauffage, la pompe P4 est en position arrêt C tant que l'une des consignes (Pc ou Tc) n'est pas approchée.

Deux situations peuvent être rencontrées:
. Pc atteinte avant Tc (phase 07)
. Tc atteinte avant Pc (phase 08)

- Si P = Pc T < Tc (phase 07), alors il faut soutirer du fluide dans la boucle, par la pompe P4, pour pouvoir constinuer à chauffer sans dépasser la pression de consigne (séquence 01 et 02 selon le sens de fonctionnement de la pompe).
- Si T = Tc P < Pc (phase 08), alors on injecte du fluide dans la boucle par la pompe P4 pour atteindre la pression de consigne Pc (séquences 01 et 02 selon le sens de fonctionnement de la pompe).

Durant cette phase, la pression dans la seconde chambre ne doit pas baisser au dessous d'une valeur-seuil fixée de façon à éviter une vaporisation du fluide. Des consignes Marche/Arrêt appropriées sont de ce fait transmises par l'automate 2 à la pompe P1 en fonction de la pression P7 mesurée.

Les phases 7 et 8 étant entièrement gérées par l'automate 2 peuvent être avantageusement intégrées à la phase 6 comme sous-séquences du processus de mise en conditions de température et de pression. Dès que ce processus est terminé, le passage à la phase suivante 09 se fait automatiquement.

### Phases d'essais

### Vieillissement en circulation (phase 09)

Le débit de circulation est porté progressivement à la valeur choisie après atteinte des consignes Pc et Tc (séquence 01). Dans la suite des opérations, de ces phases d'essais la position des vannes (L1 à L5 et M2) dépend du sens de fonctionnement de la pompe P4 et de la phase de régulation en cours (injection ou soutirage). Elles peuvent avoir par exemple la conformation des séquences 01 et 02 de la phase 07 en soutirage, ou la conformation des séquences 01 et 02 de la phase 08 en injection.

### Mesure des propriétés rhéologiques (Phase 10)

Elle est réalisée après échange du fluide à l'intérieur du viscosimètre V et isolation de celui-ci. Le remplissage du viscosimètre V comprend plusieurs étapes:
. réduction du débit dans le circuit principal
. chasse du fluide ancien dans les canalisations d'accès au viscosimètre par circulation pendant un certain temps (séquence 01)
. chasse du fluide ancien contenu dans le viscosimètre (séquence 02)
. isolation du viscosimètre et augmentation du débit dans le circuit principal (séquence 03).

La phase de mesure peut commencer à la fermeture de la vanne B4.

### Mesure des propriétés rhéologiques.

Par l'intermédiaire de la console de commande 7 l'opérateur sélectionne les paramètres conditionnant les mesures rhéologiques:
- taux de cisaillement maximum, rampe de montée en cisaillement, palier et rampe de descente en cisaillement pour les courbes rhéologiques;
- taux de cisaillement et temps de cisaillement pour les mesures de thixotropie;
- temps de gel pour les mesures "normalisées" des gels.

Puis il sélectionne le type de mesure à effectuer sur un menu et le déclenche.

En fin du cycle de mesures décidé par l'opérateur, il se produit un arrêt automatique de la rotation du rotor du viscosimètre V et une reprise de la conformation de la séquence 01 de la phase 09 avec ouverture de la vanne B4.

### Mesures de filtration dynamique (phase 11)

Le processus comporte:
- l'isolation de la réduction RS et circulation par les filtres F1 à F6 (séquence 01),
- l'établissement de la pression différentielle de filtration △P dans la pompe P3 (séquence 02),
- une séquence de filtration par ouverture sélective de la vanne G1 à G6 du filtre choisi et de la vanne V qui marque l'instant de départ de la mesure de filtration. La pression différentielle est maintenue par déplacement dans le sens S du piston de la pompe 3 (séquence 03). On mesure le volume du filtrat durant un intervalle de temps fixé ou jusqu'à ce que le volume du filtrat atteigne une limite également fixée.

Dans cette phase de mesure, on enregistre en fonction du temps: le volume filtré, le débit de circulation, la pression différentielle de filtration, et la température du fluide près du filtre sélectionné,
- l'annulation de la pression différentielle △P par compression du fluide par la pompe de régulation P4 (séquence 04).

La phase de filtration se termine par l'ouverture des vannes G1 à G6 et la reprise du vieillissement dans le circuit principal (phase 09 séquence 01).

### Mesures de filtration statique (phase 12)

Pour réaliser cette phase:
- on isole la restriction de section RS et on dérive la circulation par les filtres F1 à F6 (séquence 01) pour changer le fluide resté au repos,
- on rétablit la circulation dans le circuit principal et on établit une pression différentielle de filtration △P dans la pompe P3 (séquence 02, et
- on ouvre sélectivement la vanne G1 à G6 du filtre choisi (séquence 03).

On procède de la même façon que pour la filtration dynamique (phase 11, séquence 03) sauf que durant cette phase, le débit de circulation n'est pas enregistré. La phase de filtration statique s'achève en annulant la différence de pression △P (séquence 04), en ouvrant la vanne G concernée et en reprenant la conformation de vieillissement dans le circuit principal (phase 09, séquence 01).

### Purge de la pompe de régulation de filtration (P3) (Phase 13)

Cette opération peut se révéler nécessaire avant d'entamer une phase de filtration. La pompe est d'abord isolée (séquence 01) puis vidée partiellement par déplacement du piston (séquence 02). Dans cette séquence, la pompe P3 est placée en mode priorité au débit. Le fluide restant est ensuite comprimé, après fermeture de la vanne F, jusqu'à rétablissement de la pression (séquence 01) puis la vanne H et la vanne G concernée sont ouvertes. La conformation de vieillissement est ensuite rétablie (phase 09, séquence 01).

### Opérations de contamination du fluide

### Injection d'un fluide contaminant (Phase 14)

Durant cette phase, la pompe P4 fonctionne en mode priorité au débit et n'assurera donc pas son rôle de régulation de pression dans la boucle d'essai. Cette fonction sera assurée par la pompe P3 avec les filtres déjà utilisés. Durant cette phase, on réalise successivement:
- le déplacement du piston de la pompe P4 en butée dans le sens 1 avec transfert du fluide de la chambre 1 vers la chambre 2 (séquence 01).
- le remplissage de la chambre ch1 par déplacement du piston dans le sens 2 avec purge simultanée de la chambre Ch2 (séquence 02),
- on renouvelle les séquences 01 et 02 pour s'assurer de la qualité du fluide contaminant, avant l'injection du fluide contaminant au débit souhaité (séquence 03).

La phase 14 est précédée d'une purge de la pompe P1 et d'une partie de la tuyauterie grâce à une vanne à commande manuelle M placée entre la pompe P1 et le clapet anti-retour SAR.

### Purge du fluide contaminant en fin d'injection (Phase 15)

Après purge de la pompe P1 et d'une partie du circuit et remplacement du fluide contaminant par du fluide de forage, le reste du circuit est purgé par la pompe P4. Pour cela, la chambre 1 est remplie grâce à la pompe P1, par déplacement du piston dans le sens 2 (séquence 01). Le fluide à éliminer est ensuite transféré de la chambre Ch1 vers la chambre Ch2 (séquence 02) puis purgé avec remplissage de la chambre Ch1 par du fluide de forage (séquence 03).

Les séquences 02 et 03 sont renouvelées pour s'assurer du remplacement correct du fluide contaminant. La conformation du vieillissement est ensuite rétablie (phase 09, séquence 01).

### Débitmètre

Le débitmètre est intégré dans le circuit par intermittence lorsque les acquisitions des mesures de débit sont nécessaires. En conséquence, à partir de la phase 05, le positionnement des vannes A1, A2 est conditionné par le cycle d'acquisition des mesures de débit, à intervalles réguliers, la modification des conditions sur le régime de débit pendant l'opération de chargement du viscosimètre, par exemple, et la phase en cours (par exemple, acquisition continue pendant les opérations de filtration dynamique).

Les logiciels de commande de l'automate 2 et du micro-ordinateur MP permettent une gestion complète de l'installation. Le dispositif réalise un pilotage complet des phases et séquences successives à partir de consignes imposées par l'opérateur par l'intermédiaire du micro-ordinateur;
- il gère le traitement des données de filtration et des mesures rhéologiques et leur présentation sous la forme de courbes ou de tableau et à intervalles définis, il produit des relevés systématiques des opérations effectuées;
- il règle tous les régulateurs P.I.D. des moyens de régulation de température.

En outre il est adapté à détecter les défauts de fonctionnement et à les localiser, ce qui permet de les corriger beaucoup plus rapidement.

## Revendications

1. Dispositif pour réaliser un ensemble de mesures sur un courant de fluide circulant dans un circuit fermé (C) et soumis à certaines conditions spécifiques semblables à celles rencontrées au cours de son utilisation, comportant une pompe de circulation (P2) incluse directement dans le circuit fermé pour établir un courant de fluide dans ledit circuit, des moyens d'asservissement pour réguler la différence de pression entre la sortie et l'entrée de la pompe de circulation, des moyens (RS) pour cisailler le fluide circulant dans le circuit fermé, des moyens (D) pour mesurer les débits du fluide dans le circuit fermé, des moyens (V) pour mesurer les caractéristiques rhéologiques du fluide, des moyens de chauffage pour chauffer le fluide en circulation, des moyens de filtration (F1 à F6) du fluide en circulation, caractérisé en ce que la pompe de circulation est du type à membrane, les moyens d'asservissement sont agencés pour réguler ladite différence de pression de part et d'autre des membranes de la pompe de circulation (P2), et les moyens de chauffage sont disposés de façon à chauffer le fluide en circulation, de façon homogène, le dispositif comportant également une pompe de chargement (P1) pour introduire dans le circuit fermé un ou plusieur fluides, une pompe de régulation de filtration (P3), les moyens de filtration étant actionnés par celle-ci, une pompe d'injection et de soutirage (P4), des capteurs de mesure (CT, CP), un ensemble d'alimentation électrique (5) et un système de commande programmable connecté aux capteurs de mesure et coopérant avec l'ensemble d'alimentation électrique, pour imposer aux pompes et aux vannes de commande, des séquences opératoires pour la réalisation de l'ensemble de mesures sur le fluide en circulation.

2. Dispositif selon la revendication 1, caractérisé en ce que les moyens de chauffage comportent un ensemble de manchons chauffants régulés en température.

3. Dispositif selon la revendication 1 ou 2, caractérisé en ce que les moyens de filtration sont inclus sur un circuit dérivé (cd2) et comportent une pluralité d'unités de filtration (F1-F6) à manchons filtrants (MF) interconnectées en série, chacune d'elles étant reliée par une vanne (G1-G6) à la pompe de régulation de la filtration (P3) laquelle applique sélectivement auxdites unités de filtration une pression différentielles de part et d'autre des manchons filtrants pour dériver du fluide hors du circuit fermé.

4. Dispositif selon l'une des revendications précédentes, caractérisé en ce que le circuit (C) est réalisé essentiellement avec des portions de circuit orientées de manière à éviter la formation de dépôts susceptibles de restreindre localement la section du circuit fermé.

5. Dispositif selon l'une des revendications 1 à 4, caractérisé en ce que le système de commande programmable comporte un automate programmable (2) adapté à centraliser les valeurs des paramètres mesurés associé à un micro-ordinateur de commande (MP) pour imposer des valeurs aux paramètres de régulation et des moyens de liaison pour assurer l'interaction de l'automate et du micro-ordinateur de commande, le dispositif comportant en outre un ensemble de visualisation (6) montrant à chaque instant l'état des différents éléments du dispositif.

6. Dispositif selon la revendication 1, caractérisé en ce que la pompe de circulation (P2) est une pompe à diaphragmes métalliques connectée directement sur le circuit fermé.

7. Dispositif selon la revendication 1, caractérisé en ce que les moyens d'asservissement comportent deux capteurs de pression (CP5, CP6) disposés au voisinage respectivement de l'entrée et de la sortie de la pompe de circulation (P2) et deux capteurs de pression (CP9, CP10) disposés de part et d'autre des membranes de celle-ci.

8. Dispositif selon l'une des revendications précédentes, caractérisé en ce que la pompe de régulation de la filtration (P3) et la pompe de soutirage et d'injection (P4) sont des pompes du type à piston.

9. Dispositif selon l'une des revendications précédentes, caractérisé en ce qu'il comporte une cellule (Sc) de prélèvement de fluide dans les conditions spécifiques imposées.

10. Dispositif selon l'une des revendications précédentes, caractérisé en ce que le circuit (C) et toutes les parties métalliques en contact avec le fluide sont réalisés dans un alliage capable de résister à la corrosion par les solutions salines à haute température et à l'abrasion par des solutions chargées en particules solides.

## Patentansprüche

1. Vorrichtung zur Ausführung einer Gesamtheit von Messungen an einem in einem geschlossenen Kreis (C) zirkulierenden Fluidstrom, der gewissen spezifischen Bedingungen ähnlich denen ausgesetzt ist, die während seiner Verwendung auftreten, mit einer Zirkulationspumpe (P2), die direkt in den geschlossenen Kreis eingeschlossen ist, um einen Fluidstrom in diesem Kreis herzustellen, Regelmitteln zum Steuern der Druckdifferenz zwischen Austritt und Eintritt der Zirkulationspumpe, Mitteln (RS), um das im geschlossenen Kreis zirkulierende Fluid zu scheren, Mitteln (D) zum Messen der Durchsätze des Fluids im geschlossenen Kreis, Mitteln (V) zum Messen der rheologischen Charakteristiken des Fluids, mit Heizmitteln zum Erwärmen des in Zirkulation befindlichen Fluids, und Filtriermitteln (F1-F6) für das in Zirkulation befindliche Fluid, dadurch gekennzeichnet, daß die Zirkulationspumpe vom Membrantyp ist, die Regelmittel derart ausgebildet sind, daß sie die Druckdifferenz zu beiden Seiten der Membranen der Zirkulationspumpe (P2) regulieren, und die Heizmittel derart angeordnet sind, daß sie das in Zirkulation befindliche Fluid homogen erwärmen, wobei die Vorrichtung ebenfalls eine Ladepumpe (P1) umfasst, um in den geschlossenen Kreis eines oder mehrere Fluide einzuführen und eine Filtrierungsregulierpumpe (P3) umfasst, wobei die Filtrierungsmittel durch diese betätigt werden, eine Injektions- und Absaugpumpe (P4) vorgesehen ist sowie Meßaufnehmer (CT, CP), eine elektrische Speiseanordnung (5) und ein programmierbares Befehlssystem, das mit den Meßaufnehmern verbunden ist und mit der elektrischen Speiseanordnung zusammenwirkt, um den Pumpen und den Steuer- oder Befehlsventilen Arbeitssequenzen aufzugeben, um die Gesamtheit von Messungen am Zirkulationsfluid durchzuführen.

2. Vorrichtung nach Anspruch 1, dadurch gekennzeichnet, daß die Heizmittel eine Anordnung von temperaturgesteuerten Heizhülsen umfassen.

3. Vorrichtung nach Anspruch 1 oder 2, dadurch gekennzeichnet, daß die Filtrationsmittel in einen Parallelkreis (cd2) eingeschaltet sind und eine Vielzahl von Filtereinheiten (F1-F6) mit in Reihe geschalteten Filterhülsen (MF) umfassen, wobei jede hiervon über ein Ventil (G1-G6) mit der Filtrationsregulierpumpe (P3) vorbunden ist, welche selektiv an diese Filtrationseinheiten einen Differentialdruck zu beiden Seiten der Filterhülsen anlegen, um Fluid außerhalb des geschlossenen Kreises im Nebenschluß zu führen.

4. Vorrichtung nach einem der vorhergehenden Ansprüche, dadurch gekennzeichnet, daß der Kreis (C) im wesentlichen mit Kreisbereichen ausgestattet ist, die derart orientiert sind, daß die Bildung von Abscheidungen vermieden wird, die in der Lage sind, lokal den Querschnitt des geschlossenen Kreises einzuschnüren.

5. Vorrichtung nach einem der Ansprüche 1 bis 4, dadurch gekennzeichnet, daß das programmierbare Befehlssystem einen programmierbaren Automaten (2) umfasst, der so ausgelegt ist, daß er die Werte der gemessenen Parameter in Zuordnung zu einem Befehlsmikrorechner (MP) zentralisiert, um die Steuerparameter mit Werten zu behaften sowie Verbindungsmitteln, um die Wechselwirkung des Automaten und des Befehlsmikrorechners aufzubringen, wobei die Vorrichtung im übrigen eine Sichtbarmachungsanordnung (6) umfasst, die in jedem Augenblick den Zustand der verschiedenen Elemente der Vorrichtung anzeigt.

6. Vorrichtung nach Anspruch 1, dadurch gekennzeichnet, daß die Zirkulationspumpe (P2) eine Pumpe mit metallischen Membranen ist, die direkt am geschlossenen Kreis liegt.

7. Vorrichtung nach Anspruch 1, dadurch gekennzeichnet, daß die Regelmittel zwei Druckaufnehmer (CP5, CP6) umfassen, die benachbart jeweils dem Eintritt und dem Austritt der Zirkulationspumpe (P2) angeordnet sind sowie zwei Druckaufnehmer (CP9, CP10) zu beiden Seiten von deren Membranen angeordnet sind.

8. Vorrichtung nach einem der vorhergehenden Ansprüche, dadurch gekennzeichnet, daß die Steuerpumpe für die Filtration (P3) und die Absaug- und Injektionspumpe (P4) Pumpen vom Kolbentyp sind.

9. Vorrichtung nach einem der vorhergehenden Ansprüche, dadurch gekennzeichnet, daß sie eine Zelle (Sc) zur Entnahme von Fluid unter spezifisch vorgeschriebenen Bedingungen umfasst.

10. Vorrichtung nach einem der vorhergehenden Ansprüche, dadurch gekennzeichnet, daß der Kreis (C) und sämtliche mit dem Fluid in Kontakt kommenden metallischen Teile aus einer Legierung hergestellt sind, die in der Lage ist, korrosionsbeständig gegen Salzlösungen bei hoher Temperatur und gegen Abrieb durch mit Feststoffpartikeln beladene Lösungen zu sein.

## Claims

1. A device for carrying out a set of measurements on a flow of fluid circulating in a closed circuit (C) and subjected to certain specific conditions similar to those encountered during use, having a circulation pump (P2) directly integrated in the closed circuit to establish a flow of fluid in the circuit, control means to regulate the pressure differential between the outlet and inlet of the circulation pump, means (RS) for shearing the fluid circulating in the closed circuit, means (D) for measuring the fluid flow rates in the closed circuit, means (V) for measuring the rheological characteristics of the fluid, heating means for heating the circulating fluid, means (F1 to F6) for filtering the circulating fluid, characterised in that the circulation pump is of the diaphragm type, the control means are set so as to regulate the difference in pressure on either side of the diaphragms of the circulation pump (P2), and the heating means are arranged so as to heat the circulating fluid homogeneously, the device also having a supply pump (P1) for bringing one or several fluids into the closed circuit, a filter regulating pump (P3), the filtering means being actuated by this pump, an injection and extraction pump (P4), measuring sensors (CT, CP), an electricity supply unit (5) and a programmable control unit connected to the measuring sensors and co-operating with the electricity supply unit to assign operating sequences to the pumps and control valves that will carry out the set of measurements on the circulating fluid.

2. A device in accordance with claim 1, characterised in that the heating means have a set of temperature-controlled heating sleeves.

3. A device in accordance with claim 1 or 2, characterised in that the filtering means are integrated in a by-pass circuit (cd2) and have a plurality of filtering units (F1-F6) with filtering sleeves (MF) inter-connected in series, each of these being connected via a valve (G1-G6) to the filter regulating pump (P3) which applies differential pressure selectively to the filtering units on either side of the filtering sleeves to divert the fluid out of the closed circuit.

4. A device in accordance with one of the previous claims, characterised in that the circuit (C) is mainly made up of circuit sections oriented in such a way as to prevent the formation of deposits that might locally obstruct the closed circuit section.

5. A device in accordance with one of claims 1 to 4, characterised in that the programmable control system has a programmable automaton (2) programmed to centralise the values of the parameters measured, associated with a controlling micro-processor (MP) for assigning values to the regulating parameters and liaison means to ensure that there is inter-action between the automaton and the controlling micro-processor, the device also having a visual display unit (6) showing the status of the various elements of the device at any time.

6. A device in accordance with claim 1, characterised in that the circulation pump (P2) is a pump with metallic diaphragms directly connected to the closed circuit.

7. A device in accordance with claim 1, characterised in that the control means have two pressure sensors (CP5, CP6) located respectively near the inlet and the outlet of the circulation pump (P2) and two pressure sensors (CP9, CP10) located on either side of the pump diaphragms.

8. A device in accordance with one of the previous claims, characterised in that the filter regulating pump (P3) and the injection and extraction pump (P4) are piston type pumps.

9. A device in accordance with one of the previous claims, characterised in that it has a cell (Sc) for drawing off fluid under specific given conditions.

10. A device in accordance with one of the previous claims, characterised in that the circuit (C) and all the metallic parts in contact with the fluid are made from an alloy capable of withstanding corrosion by saline solutions at high temperature and abrasion by solutions carrying solid particles.
